# EUROPEAN PATENT APPLICATION

(11) **EP 4 144 299 A1**
(43) Date of publication of application: **08.03.2023**
(21) Application number: 21795879.2
(22) Date of filing: 21.04.2021
(51) Int. Cl.: A61B 7/04, A61B 3/12, A61B 5/02

(54) **MEDICAL SYSTEM AND MEDICAL INFORMATION PROCESSING DEVICE**

(30) Priority: 01.05.2020 JP 2020081482
(71) Applicant: RIKEN, Wako-shi, Saitama 351-0198 (JP); TOPCON CORPORATION, Tokyo 174-8580 (JP)
(72) Inventor: WADA, Satoshi, Wako-shi, Saitama 351-0198 (JP); MARUYAMA, Masayuki, Wako-shi, Saitama 351-0198 (JP); SAITO, Norihito, Wako-shi, Saitama 351-0198 (JP); TANEISHI, Kei, Wako-shi, Saitama 351-0198 (JP); FUKUMA, Yasufumi, Tokyo 174-8580 (JP); AKIBA, Masahiro, Tokyo 174-8580 (JP); TAKEUCHI, Gaku, Tokyo 174-8580 (JP); MINAMIDE, Kana, Tokyo 174-8580 (JP)
(74) Representative: Gagel, Roland
(86) International application number: PCT/JP2021/016179
(87) International publication number: WO 2021/220911

(57) **Abstract**

A medical system 1 according to an exemplary embodiment includes a data acquisition unit 10 and a data processing unit 20. The data acquisition unit 10 is configured so as to acquire, from a patient, at least two types of data among blood oxygen data, auscultatory sound data, ocular image data, and ocular blood flow data. The data processing unit 20 is configured so as to process the at least two types of data acquired by the data acquisition unit 10, in order to detect a change in the condition of the circulatory system that accompanies an infectious disease.

## Description

### TECHNICAL FIELD

The present disclosure relates generally to a medical system and a medical information processing apparatus.

### BACKGROUND OF THE INVENTION

Symptoms of a disease and signs of aggravation (exacerbation, worsening) of a disease are complex, and various techniques and technologies have been developed to detect them. For example, Patent Document 1 below discloses, as a technique or technology for determining the risk of infection without using advanced medical knowledge, a technique or technology for determining the risk of infection on the basis of the presence or absence of abnormalities in arterial blood oxygen saturation, body temperature, and heart rate.

### PRIOR ART DOCUMENTS

### PATENT DOCUMENTS

PATENT DOCUMENT 1: Japanese Unexamined Patent Application Publication No. 2016-123605

### BRIEF SUMMARY OF THE INVENTION

### PROBLEM TO BE SOLVED BY THE INVENTION

One object of the present disclosure is to provide a novel technique or technology for detecting a symptom associated with an infectious disease and/or a sign of aggravation with a high degree of precision.

### MEANS FOR SOLVING THE PROBLEM

A medical system according to some aspect examples includes: a data acquiring unit configured to acquire from a patient two or more types of data among blood oxygen saturation data, auscultatory sound data, ocular image data, and ocular blood flow data; and a data processor configured to process the two or more types of data acquired by the data acquiring unit to detect a conditional change in a circulatory system associated with an infectious disease.

In a medical system according to some aspect examples, the conditional change in the circulatory system may include one or more of an onset of pneumonia, a change in a condition of pneumonia, an onset of hypoxemia, a change in a condition of hypoxemia, and a change in a condition of cerebral blood flow.

In a medical system according to some aspect examples, the ocular image data may include fundus image data depicting an eye fundus of the patient.

In a medical system according to some aspect examples, the data processor may be configured to detect the conditional change in the circulatory system based on a running pattern of a blood vessel depicted in the fundus image data.

In a medical system according to some aspect examples, the fundus image data may include one or more types of image data among fundus camera image data, slit lamp image data, optical coherence tomography image data, and scanning laser image data.

In a medical system according to some aspect examples, the ocular image data may include color fundus image data depicting an eye fundus of the patient.

In a medical system according to some aspect examples, the data processor may be configured to detect the conditional change in the circulatory system based on color information in the color fundus image data.

In a medical system according to some aspect examples, the color fundus image data may include one or more types of image data among fundus camera image data, slit lamp image data, and scanning laser image data.

In a medical system according to some aspect examples, the data processor may include an inference processor configured to perform inference processing that derives information on the conditional change in the circulatory system from the ocular image data acquired by the data acquiring unit by using a trained model constructed by machine learning using training data that includes ocular image data and diagnostic result data.

In a medical system according to some aspect examples, the ocular blood flow data may include fundus blood flow data that represents blood flow dynamics in an eye fundus blood vessel of the patient.

In a medical system according to some aspect examples, the fundus blood flow data may include waveform data that represents a time series change in blood flow velocity in an eye fundus artery of the patient, and the data processor may be configured to detect the conditional change in the circulatory system based on the waveform data.

In a medical system according to some aspect examples, the fundus blood flow data may include two or more pieces of waveform data respectively acquired from the patient in two or more different time periods, and the data processor may be configured to detect the conditional change in the circulatory system by comparing the two or more pieces of waveform data.

In a medical system according to some aspect examples, the fundus blood flow data may include either one of or both blood flow velocity data and blood flow rate data for the eye fundus artery of the patient, and the data processor may be configured to detect the conditional change in the circulatory system based on either one of or both the blood flow velocity data and the blood flow rate data.

In a medical system according to some aspect examples, the data acquiring unit may include an optical coherence tomography apparatus configured to perform scanning of an eye fundus of the patient to acquire the fundus blood flow data.

In a medical system according to some aspect examples, the auscultatory sound data may include lung sound data acquired from the patient by an electronic stethoscope.

In a medical system according to some aspect examples, the data acquiring unit may be configured to further acquire either one of or both body temperature data and heart rate data from the patient.

A medical system according to some aspect examples may further include a transmitter configured to transmit information output from the data processor to a doctor's computer terminal located remotely from the data acquiring unit.

A medical system according to some aspect examples may further include an operation unit used for performing a remote operation of the data acquiring unit.

A medical information processing apparatus of some aspect examples includes: a data receiving unit configured to receive two or more types of data among blood oxygen saturation data, auscultatory sound data, ocular image data, and ocular blood flow data acquired from a patient; and a data processor configured to process the two or more types of data received by the data receiving unit to detect a conditional change in a circulatory system associated with an infectious disease.

In a medical information processing apparatus of some aspect examples, the conditional change in the circulatory system may include one or more of an onset of pneumonia, a change in a condition of pneumonia, an onset of hypoxemia, a change in a condition of hypoxemia, and a change in a condition of cerebral blood flow.

In a medical information processing apparatus of some aspect examples, the ocular image data may include fundus image data depicting an eye fundus of the patient.

In a medical information processing apparatus of some aspect examples, the data processor may be configured to detect the conditional change in the circulatory system based on a running pattern of a blood vessel depicted in the fundus image data.

In a medical information processing apparatus of some aspect examples, the ocular image data may include color fundus image data depicting an eye fundus of the patient.

In a medical information processing apparatus of some aspect examples, the data processor may be configured to detect the conditional change in the circulatory system based on color information in the color fundus image data.

In a medical information processing apparatus of some aspect examples, the data processor may include an inference processor configured to perform inference processing that derives information on the conditional change in the circulatory system from the ocular image data received by the data receiving unit by using a trained model constructed by machine learning using training data that includes ocular image data and diagnostic result data.

In a medical information processing apparatus of some aspect examples, the ocular blood flow data may include fundus blood flow data that represents blood flow dynamics in an eye fundus blood vessel of the patient.

In a medical information processing apparatus of some aspect examples, the fundus blood flow data may include waveform data that represents a time series change in blood flow velocity in an eye fundus artery of the patient, and the data processor may be configured to detect the conditional change in the circulatory system based on the waveform data.

In a medical information processing apparatus of some aspect examples, the fundus blood flow data may include two or more pieces of waveform data respectively acquired from the patient in two or more different time periods, and the data processor may be configured to detect the conditional change in the circulatory system by comparing the two or more pieces of waveform data.

In a medical information processing apparatus of some aspect examples, the fundus blood flow data may include either one of or both blood flow velocity data and blood flow rate data for the eye fundus artery of the patient, and the data processor may be configured to detect the conditional change in the circulatory system based on either one of or both the blood flow velocity data and the blood flow rate data.

In a medical information processing apparatus of some aspect examples, the auscultatory sound data may include lung sound data.

A medical information processing apparatus of some aspect examples further may include the data receiving unit further receives either one of or both body temperature data and heart rate data acquired from the patient.

A medical information processing apparatus of some aspect examples may further include a transmitter configured to transmit information generated by the data processor to a doctor's computer terminal located remotely from at least one apparatus used to acquire the two or more types of data from the patient.

### EFFECT OF THE INVENTION

These aspect examples allow the precision of detection processing of disease symptoms associated with an infectious disease and/or signs of aggravation to be improved.

### BRIEF DESCRIPTION OF THE SEVERAL VIEWS OF THE DRAWING

FIG. 1 is a diagram illustrating an example of a configuration of a medical system according to an aspect example.
FIG. 2 is a diagram illustrating an example of a configuration of data to be processed by a medical system according to an aspect example.
FIG. 3 is a diagram illustrating an example of a configuration of a medical system according to an aspect example.
FIG. 4 is a diagram illustrating an example of a configuration of a medical system according to an aspect example.
FIG. 5 is a diagram illustrating an example of a configuration of a medical system according to an aspect example.
FIG. 6 is a flowchart illustrating an example of an operation of a medical system according to an aspect example.
FIG. 7 is a diagram illustrating an example of a configuration of a medical information processing apparatus according to an aspect example.

### DETAILED DESCRIPTION OF THE INVENTION

The present disclosure provides descriptions of several aspect examples of a medical system and a medical information processing apparatus. Some aspect examples are configured to detect a change in a condition (state) of a circulatory system (a conditional change in a circulatory system) associated with an infectious disease by processing using a computer two or more types of data freely selected from a set including blood oxygen saturation data, auscultatory sound data, ocular image data, and ocular blood flow data. This computer processing may include diagnostic inference. This diagnostic inference may be performed, for example, by using one of an algorithm that uses a trained model constructed by machine learning (inference model) and an algorithm that does not use such a trained model. Alternatively, the diagnostic inference may be performed by a combination of these two types of algorithms.

The types of data subjected to the computer processing are not limited to blood oxygen saturation data, auscultatory sound data, ocular image data, and ocular blood flow data, and may include other types of data in some examples. Examples of these other types of data may include biological data acquired from a patient using an examination apparatus, data stored in a storage device such as a database, and so forth. Here, examples of the biological data may include body temperature data, heart rate data, and so forth, and examples of the data stored in the storage device may include electronic medical record data, clinical encounter (patient encounter, medical interview) data, patient's background information, and so forth. Examples of the patient's background information may include age, treatment history, medical history, health history, medication history, surgical history, and so forth.

Some aspect examples may be configured to be capable of detecting complex physiological events such as symptoms of a disease associated with an infection and signs of aggravation of a disease with high precision, by performing comprehensive processing of blood oxygen saturation data, auscultatory sound data, ocular image data, ocular blood flow data. In particular, such aspect examples may be configured to be capable of detecting a conditional change in a circulatory system associated with an infectious disease with high precision. In addition to this, some aspect examples are devised by taking the background described below into account, and can achieve corresponding advantageous effects.

Healthcare workers such as doctors and nurses are exposed to the risk of in-hospital infection (healthcare-associated infection). For example, during the pandemic of the novel coronavirus infection (Coronavirus Disease 2019; COVID-19) in 2020, the risk of infection to healthcare workers became one of major problems, as cluster infections occurred at medical institutions that were crowded with patients. The increased risk of infection to healthcare workers can occur not only during epidemics of infectious diseases, but also in the event of disasters or major accidents.

In general, it is considered important to ensure a sufficient distance between people (what is referred to as social distancing) to reduce the risk of infection. However, it is not easy to keep adequate social distances in standard clinical environment. For example, when using a stethoscope to listen to sounds generated by a heart, lungs, blood vessels, and so forth, or when using a slit lamp microscope to observe an eye, the doctor or other healthcare workers must be in close proximity to the patient to carry out the procedure.

Some aspect examples may be configured to provide a result obtained by the above-mentioned processing performed on the basis of the two or more types of data, to a doctor's computer terminal that is located remotely from an apparatus that has used for acquisition of the two or more types of data from the patient. Here, the apparatus used for the acquisition of the two or more types of data may be a data processor or an examination apparatus. Further, some example aspects may be configured to perform an operation of a data processor (or an examination apparatus) from a remote location. Such a configuration makes it possible to realize utilization, for a diagnostic purpose, of data acquired from examinations (e.g., auscultation, slit lamp examination, etc.) that could not previously be conducted without being in close proximity to a patient. In other words, according to these aspect examples, it becomes possible to maintain social distances between patients and healthcare workers, and also to achieve detection of complex physiological events, such as disease symptoms and aggravation signs, with high precision.

Here, the "remote location" or being "located remotely" may be any positional relationship that can keep social distances between patients and healthcare workers. For example, the doctor's computer terminal may be located in a room separate from a room in which the examination apparatus is located, or may be located in a facility separate from a facility in which the examination apparatus is located. In addition, a device used for conducting an operation of the examination apparatus from a remote location, such as an operation device and an operation unit, may be located in a room separate from a room in which the examination apparatus is located, or may be located in a facility separate from a facility in which the examination apparatus is located.

It should be noted that in the cases where an examination is conducted under an adequate infection protection system, such as when full protective clothing is worn, it is not necessary to ensure social distancing. In the cases where only some of the examinations (referred to as specific examinations) are conducted under an adequate infection protection system, some aspect examples may be configured to provide information to a doctor's computer terminal located remotely from each of the examination apparatuses used for examinations other than the specific examinations.

Any of the aspect examples in the present disclosure may be modified by any of the matters and items described in the documents cited herein and any other known techniques or technologies. This modification may be, for example, any of addition, combination, substitution, replacement, deletion, omission, and other processing.

At least one or more of the functions of the elements described in the present disclosure may be implemented by using a circuit configuration (circuitry) or a processing circuit configuration (processing circuitry). The circuitry or the processing circuitry may include any of the followings, all of which are configured and/or programmed to execute at least one or more functions disclosed herein: a general purpose processor, a dedicated processor, an integrated circuit, a central processing unit (CPU), a graphics processing unit (GPU), an application specific integrated circuit (ASIC), a programmable logic device (e.g., a simple programmable logic device (SPLD), a complex programmable logic device (CPLD), or a field programmable gate array (FPGA)), an existing or conventional circuit configuration or circuitry, and any combination of these. A processor may be considered to be processing circuitry or circuitry that includes a transistor and/or another circuitry. In the present disclosure, circuitry, a circuit, a computer, a processor, a unit, a means, a part, a section, or a term similar to these may include hardware that executes at least one or more functions disclosed herein, and/or hardware that is programmed to execute at least one or more functions disclosed herein. Hardware may be the hardware disclosed herein, or alternatively, known hardware that is programmed and/or configured to execute at least one or more functions described herein. In the case in which the hardware is a processor, which may be considered as a certain type of circuitry, then circuitry, a circuit, a computer, a processor, a unit, a means, a part, a section, or a term similar to these may be a combination of hardware and software. In this case, the software may be used to configure the hardware and/or the processor.

The aspect examples described below may be freely combined. For example, two or more of the aspect examples may be combined at least in part.

### < Configuration of Medical System >

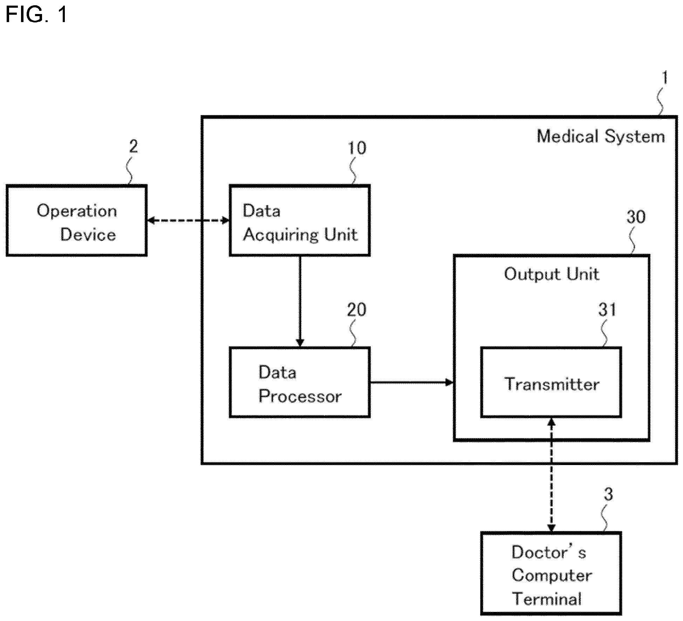

Several examples of a configuration of a medical system of some aspect examples will be described. The medical system 1 illustrated in FIG. 1 includes the data acquiring unit 10, the data processor 20 and the output unit 30. The medical system 1 may further include the operation device 2.

In some typical examples, the data acquiring unit 10 and the data processor 20 are connected via a communication line. This communication line may form a network in a medical institution, or may form a network across a plurality of facilities. The communication technology applied to this communication line may be freely determined, and may include any of various kinds of known communication technologies such as wired communication, wireless communication, near field communication, and so forth. The aspect or mode of connection between the data processor 20 and the output unit 30 may also be the same. In some alternative examples, the data processor 20 and the output unit 30 may be individual functional units installed in the same computer.

The operation device 2 is used by a healthcare worker to perform an operation of the data acquiring unit 10 (examination apparatus) from a remote location (i.e., to perform remote operation, remote manipulation, or remote control of the data acquiring unit 10). The operation device 2 is also used by a healthcare worker (examiner) to provide an instruction and so forth to a patient (subject) who is undergoing an examination using the data acquiring unit 10 (examination apparatus). The operation device 2 of some examples includes any of a computer, an operation panel, and so forth.

The data acquiring unit 10 is configured to acquire ophthalmic data from a patient. In particular, the data acquiring unit 10 is configured to acquire from a patient two or more types of data among blood oxygen saturation data, auscultatory sound data, ocular image data, and ocular blood flow data.

An apparatus used for acquisition of blood oxygen saturation data from a patient (referred to as a blood oxygen saturation measurement apparatus) may be, for example, a pulse oximeter described in Japanese Unexamined Patent Application Publication No. 2019-111010. The type of blood oxygen saturation data detected by the blood oxygen saturation measurement apparatus may be freely determined, and may be, for example, at least one of: oxygen saturation data, oxygen content data (oxygen concentration data), and oxygen feed rate data.

An apparatus used for acquisition of auscultatory sound data from a patient (referred to as an auscultatory sound measurement apparatus) may be, for example, an electronic stethoscope described in Japanese Unexamined Patent Application Publication No. 2017-198. The type of auscultatory sound detected by the use of the auscultatory sound measurement apparatus may be freely determined, and may be, for example, at least one of: tracheal breath sounds data, bronchial breath sounds data, alveolar breath sounds data (lung sounds data), heart sounds data, and blood flow sounds data.

An apparatus used for acquisition of ocular image data from a patient (referred to as an ophthalmic imaging apparatus) may be any ophthalmic modality apparatus. The ophthalmic modality employable as the ophthalmic imaging apparatus may be, for example, a photographing type modality or a scanning type modality. Types of the ophthalmic imaging apparatus include an optical coherence tomography apparatus, a fundus camera, a slit lamp microscope, and a surgical microscope. Further, the ophthalmic imaging apparatus may be a fundus imaging apparatus capable of acquiring fundus image data. Fundus image data is, for example, image data in which a fundus blood vessel is depicted, and is used for analysis of a blood vessel running pattern.

The optical coherence tomography apparatus and/or the fundus camera may be, for example, apparatuses in which various kinds of imaging preparation operations are automated, as described in Japanese Unexamined Patent Application Publication No. 2020-44027. Note that the imaging preparation operations are performed to prepare imaging conditions. Examples of the imaging preparation operations include alignment adjustment, focus adjustment (focusing), optical path length adjustment, polarization adjustment, and light amount adjustment. Further, the optical coherence tomography apparatus and/or the fundus camera may be configured to perform an automatic operation for maintaining favorable imaging conditions achieved by the imaging preparation operations. Examples of the automatic operation include automatic alignment adjustment in accordance with an eye movement (eye tracking), automatic optical path length adjustment in accordance with an eye movement (Z lock), and so forth. These automatic operations are effective, for example, in examinations that are conducted without an examiner by the side of a subject.

Ocular image data acquired by the optical coherence tomography apparatus (referred to as optical coherence tomography image data) may be, for example, at least one of: three dimensional image data obtained by applying three dimensional scanning to a fundus, projection image data of three dimensional image data, and optical coherence tomography angiography (OCTA) image data.

Ocular image data acquired by the fundus camera (referred to as fundus camera image data) may be, for example, at least one of: color fundus image data, infrared fundus image data, and fluorescent contrast fundus image data (e.g., fluorescein angiography image data, indocyanine green angiography image data, or the like).

A scanning laser ophthalmoscope may be, for example, an apparatus described in Japanese Unexamined Patent Application Publication No. 2014-226156. Ocular image data acquired by the scanning laser ophthalmoscope may be, for example, at least one of: color fundus image data, monochrome fundus image data, and fluorescent contrast fundus image data.

A slit lamp microscope may be, for example, an apparatus usable for remote imaging, as that described in Japanese Unexamined Patent Application Publication No. 2019-213734. Ocular image data acquired by the slit lamp microscope may be, for example, at least one of: color fundus image data, anterior eye segment cross sectional image data, and anterior eye segment three dimensional image data.

Any known apparatuses may be employed for ophthalmic imaging apparatuses of other types than those described above. For example, a surgical microscope usable for remote surgery, as that described in Japanese Unexamined Patent Application Publication No. 2002-153487, may be employed as an ophthalmic imaging apparatus.

An apparatus used for acquisition of ocular blood flow data from a patient (referred to as an ocular blood flow measurement apparatus) may be an apparatus of any measurement method or technique. The ocular blood flow data includes, for example, data that represents blood flow dynamics in a fundus blood vessel of the patient (referred to as fundus blood flow data). The ocular blood flow measurement apparatus may be, for example, an optical coherence tomography (OCT) apparatus described in Japanese Unexamined Patent Application Publication No. 2019-54994, an OCT apparatus described in Japanese Unexamined Patent Application Publication No. 2020-48730, or the like. An ocular blood flow measurement apparatus according to some aspect examples may be configured to utilize the known techniques or technologies described above, thereby acquiring waveform data that represents a time series change (time course change, chronological change, time-dependent change) in blood flow velocity in a fundus artery of a patient, and/or, acquiring blood flow velocity data and/or blood flow rate data (blood flow amount data) for a fundus artery of a patient. In some typical examples, the waveform data is a time series change graph of blood flow velocity represented by a two dimensional coordinate system in which the horizontal axis represents time and the vertical axis represents blood flow velocity. An apparatus employable as an ocular blood flow measurement apparatus is not limited to an optical coherence tomography apparatus. For example, a laser speckle flowgraphy (LSFG) apparatus described in Japanese Unexamined Patent Application Publication (Translation of PCT Application) No. 2017-504836 may also be employed as an ocular blood flow measurement apparatus.

As mentioned above, the types of data acquired by the data acquiring unit 10 of some aspect examples are not limited to blood oxygen saturation data, auscultatory sound data, ocular image data, and ocular blood flow data. In some aspect examples, the data acquiring unit 10 may be capable of acquiring body temperature data and/or heart rate data. Body temperature data of a patient is acquired, for example, by using the thermometer in the system described in Japanese Unexamined Patent Application Publication No. 2018-29964. Heart rate data of a patient (e.g., heart rate, electrocardiographic waveform, etc.) is acquired, for example, by using the heart rate and electrocardiogram meter described in Japanese Unexamined Patent Application Publication No. 2017-148577.

Some aspect examples may be capable of acquiring any type of data such as blood flow data, pulse data (pulse rate data), respiratory function data, blood pressure data, and so forth. For example, blood flow data (e.g., blood flow velocity data, blood flow rate data, blood flow velocity distribution data, etc.) and/or pulse data (e.g., pulse rate, the number of pulses, etc.) may be acquired by using any of the following devices: the ultrasonic blood flowmeter described in Japanese Unexamined Patent Application Publication No. 2008-36095, the laser blood flowmeter described in Japanese Unexamined Patent Application Publication No. 2008-154804, and the electromagnetic blood flowmeter described in Japanese Unexamined Patent Application Publication No. Hei10-328152. In addition, respiratory function data may be acquired by using the respiratory monitoring system described in Japanese Unexamined Patent Application Publication (Translation of PCT Application) No. 2019-527117. Here, examples of the respiratory function data include respiratory rate data (breathing rate data), tidal volume data, minute ventilation data (pulmonary ventilation per minute data, minute volume (MV) data, minute breathing capacity (MBC) data), intratracheal pressure data, airflow velocity and amount data, ventilation work amount data, inspiratory gas concentration data, and inspiratory water vapor data. Further, blood pressure data (e.g., blood pressure value, etc.) and pulse data (e.g., pulse rate, etc.) may be acquired by using the blood pressure meter in the system described in Japanese Unexamined Patent Application Publication No. 2018-29964.

Some aspect examples may be configured to execute processing on ocular characteristic data (ocular feature data, ocular property data) acquired by an ophthalmic measurement apparatus. Ocular characteristic data is data indicating a condition of an eye, and is characteristic data such as numerical data, evaluation data, or the like. Types of the ophthalmic measurement apparatus include, in addition to the ocular blood flow measurement apparatus described above, an ocular refraction measurement apparatus, a tonometer, a corneal endothelial cell examination apparatus (a specular microscope), a higher order aberration measurement apparatus (a wavefront analyzer), a visual acuity test apparatus, a perimeter, a microperimeter, an ocular axial length measurement apparatus, an electroretinography apparatus, a binocular visual function examination apparatus, a color vision examination apparatus, and so forth. Japanese Unexamined Patent Application Publication No. 2018-38518 discloses an example of the ocular refraction measurement apparatus (a refractometer, a keratometer), an example of the tonometer (a non-contact tonometer), an example of the specular microscope, and an example of the wavefront analyzer. The visual acuity test apparatus may be, for example, an apparatus capable of conducting remote visual acuity tests described in Japanese Unexamined Patent Application Publication No. 2018-110687. Any known apparatuses may be employed for other types of ophthalmic measurement apparatuses.

In the present aspect, at least one of the examination apparatuses included in the data acquiring unit 10 (e.g., an electronic stethoscope, an ophthalmic imaging apparatus, an ophthalmic measurement apparatus, etc.) may be capable of performing remote operation and/or remote control.

As an example, considering the risk of infection to healthcare workers, separate rooms for examination and operation can be prepared as an examination room and an operation room. Here, the examination room is used for conducting an examination using an examination apparatus, and the operation room is used for performing an operation of the examination apparatus. In addition to the examination apparatus, the examination room may be equipped with any of the following types of devices: a speaker and/or a display to output an instruction issued by an operator from the operation room (e.g., voice, image, video, etc.), a video camera to photograph a subject (patient) in the examination room, a microphone to input voice of the subject, and a computer connected to the examination apparatus.

On the other hand, the operation room is equipped with the operation device 2 used for performing remote operation of the examination apparatus. The operation device 2 includes any one or more of a computer, an operation panel, a display, a video camera, and a microphone. The computer executes processing for remote control. The computer is connected to the examination apparatus installed in the examination room. The operation panel, the video camera, and the microphone are used to input an instruction for the subject. The display shows visualization of data acquired by the examination apparatus, visualization of information for remote operation (e.g., a screen, information from the examination room, etc.), and so forth.

With such configurations, the operator (healthcare worker) can conduct remote operation, from the operation room, of the examination apparatus located in the examination room using an application programming interface (API), for example. The operator can also send an instruction from the operation room to the subject by the use of a videophone. As a result, the subject can undergo an examination by him/herself in accordance with the instruction issued by the operator at a remote location. This makes it possible to significantly reduce the risk of the operator getting an infection from the subject.

In order to more preferably conduct an examination carried out by a patient (subject) by him/herself, the above-mentioned examination apparatus with automated preparation operations may be used. In this case, it is conceivable that an examination can be performed without requiring an instruction from an operator. In some cases, no assistant (such as an operator) may be prepared. However, some patients may find it difficult to perform an examination alone. Therefore, the following measures may be taken, for example: an assistant may be arranged on standby at a remote location, or an assistant may be arranged to monitor the status, state, condition, situation, or the like of an examination from a remote location. The assistant (e.g., operator, etc.) who sends an instruction to the patient may be a personified computer system or an anthropomorphic computer system. A typical example of such computer systems is an automatic response system configured by using artificial intelligence technology.

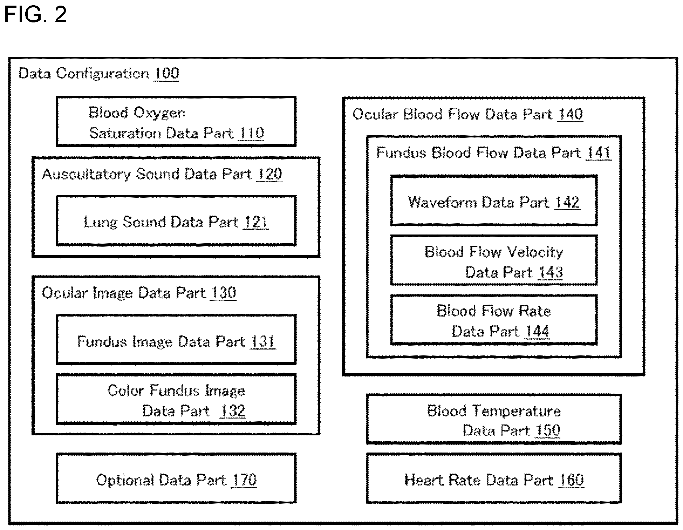

FIG. 2 shows an example of data configuration for processing (e.g., recording, transmitting, etc.) various kinds of data acquired by the data acquiring unit 10. The data configuration 100 of the present example includes the blood oxygen saturation data part 110, the auscultatory sound data part 120, the ocular image data part 130, the ocular blood flow data part 140, the body temperature data part 150, the heart rate data part 160, and the optional data part 170.

The blood oxygen saturation data part 110 is a region (e.g., folder, etc.) in which blood oxygen saturation data acquired by a blood oxygen saturation measurement apparatus in the data acquiring unit 10 is recorded.

The auscultatory sound data part 120 is a region in which auscultatory sound data acquired by an auscultatory sound measurement apparatus in the data acquiring unit 10 is recorded.

The auscultatory sound data part 120 includes the lung sound data part 121. The lung sound data part 121 is a region in which lung sound data acquired by an auscultatory sound measurement apparatus is recorded. Note that types of sub-regions provided in the auscultatory sound data part 120 are not limited to the lung sound data part 121, and may include a region in which a freely selected type of auscultatory sound data is recorded.

The ocular image data part 130 is a region in which ocular image data acquired by an ophthalmic imaging apparatus in the data acquiring unit 10 is recorded. The ocular image data part 130 includes the fundus image data part 131 and the color fundus image data part 132.

In the fundus image data part 131, for example, image data to be used for analysis of a running pattern of a fundus blood vessel or for other types of processing is recorded. Examples of fundus image data recorded in the fundus image data part 131 include the following types of data: optical coherence tomography image data (e.g., three dimensional image data, projection image data, optical coherence tomography angiography image data, etc.), fundus camera image data (e.g., color fundus image data, infrared fundus image data, fluorescent contrast fundus image data, etc.), scanning laser image data (e.g., color fundus image data, monochrome fundus image data, fluorescent contrast fundus image data, etc.), color fundus image data acquired by a slit lamp microscope, and so forth.

In the color fundus image data part 132, for example, image data to be used for analysis of color tones of an eye fundus or for other types of processing is recorded. Examples of fundus image data recorded in the color fundus image data part 132 include the following types of data: fundus camera image data (color fundus image data), scanning laser image data (color fundus image data), color fundus image data acquired by a slit lamp microscope, and so forth.

In the case where the same image data is used for both blood vessel running pattern analysis and color tone analysis, there is no need to record this image data in both the fundus image data part 131 and the color fundus image data part 132. For example, such image data may be recorded in one of the fundus image data part 131 and the color fundus image data part 132, as well as information (e.g., a tag, etc.) indicating that this image data can also be used for another purpose corresponding to the other of the fundus image data part 131 and the color fundus image data part 132 may be attached to this image data. According to such a configuration, it becomes possible to use a single piece of image data for both purposes.

The ocular blood flow data part 140 is a region in which ocular blood flow data acquired by an ocular blood flow measurement apparatus in the data acquiring unit 10 is recorded. The ocular blood flow data part 140 includes the fundus blood flow data part 141. The fundus blood flow data part 141 records data representing blood flow dynamics in a fundus blood vessel, which is referred to as fundus blood flow data as mentioned above.

The fundus blood flow data part 141 includes the waveform data part 142, the blood flow velocity data part 143, and the blood flow rate data part 144. In the waveform data part 142, recorded is waveform data that represents a time series change in blood flow velocity acquired by blood flow measurement applied to a fundus artery. In the blood flow velocity data part 143, recorded is blood flow velocity data acquired by blood flow measurement applied to a fundus artery. In the blood flow rate data part 144, recorded is blood flow rate data acquired by blood flow measurement applied to a fundus artery.

Note that, waveform data is generated from a plurality of pieces of blood flow velocity data acquired respectively at a plurality of different time points, blood flow velocity data at a specific time point is obtained from waveform data, and blood flow rate data is calculated from waveform data, blood flow velocity data, or like type of data. Considering these facts, it is not necessary to provide all the waveform data part 142, the blood flow velocity data part 143, and the blood flow rate data part 144. Some typical examples may be configured to record only a specific type of data that is provided to processing performed by the data processor 20 in the ocular blood flow data part 140. Some alternative examples may be configured to record only waveform data in the ocular blood flow data part 140, and obtain blood flow velocity data, blood flow rate data, etc. from this waveform data by the use of the data processor 20.

The body temperature data part 150 is a region in which body temperature data acquired by a thermometer in the data acquiring unit 10 is recorded. The heart rate data part 160 is a region in which heart rate data acquired by a heart rate meter (or an electrocardiogram meter) in the data acquiring unit 10 is recorded.

The optional data part 170 is a region in which a freely selected type of data is recorded. For example, in the optional data part 170, data acquired by an examination apparatus, other than those mentioned above, in the data acquiring unit 10 may be recorded. Further, electronic medical record data, clinical encounter (patient encounter, medical interview) data, and so forth may be recorded in the optional data part 170. Furthermore, information on a patient (subject) may be recorded in the optional data part 170. Examples of such patient information include an identifier, patient's background information, and so forth.

The data processor 20 performs various kinds of data processing. The data processor 20 of the present aspect example is configured to process data acquired by the data acquiring unit 10 in order to detect a conditional change in a circulatory system associated with an infectious disease.

Examples of conditions (e.g., diseases, symptoms, physiological events, etc.) of a circulatory system associated with an infectious disease include pneumonia, hypoxemia, and circulatory dysfunction (e.g., cerebral blood flow abnormalities, etc.) associated with novel coronavirus infection (COVID-19). The present aspect example is configured to be capable of detecting a change in at least one of these conditions.

According to the Journal of the Japanese Society of Internal Medicine, Vol. 109, pp. 392-395 (2020), the severity distribution of novel coronavirus infection (COVID-19) shows the following results: mild 80.9%, moderate 13.8%, severe 4.7%, and unknown 0.6%. Here, mild refers to no pneumonia to mild pneumonia, moderate refers to dyspnea, respiratory rate ≥ 30 breaths/min, oxygen saturation (SpO2) ≤ 93%, or rapid deterioration of a lung shadow, and severe refers to any of the respiratory failure, shock, and multiple organ failure. With reference to the severity distribution, it can be thought that oxygen saturation decreases with the progression of hypoxemia associated with novel coronavirus infection (COVID-19) (hypoxemia associated with pneumonia). Against such a background, the present aspect example may be configured to employ blood oxygen saturation data, which has been acquired by a blood oxygen saturation measurement apparatus in the data acquiring unit 10, as an evaluation index (assessment index) for hypoxemia associated with novel coronavirus infection (COVID-19). The same may apply to other types of infectious diseases.

The following case report of the Japanese Association for Infectious Diseases reports a case in which a marked decrease in oxygen saturation and temperature rise were not observed in a patient with novel coronavirus infection (COVID-19) during follow-up hospitalization of another disease: "A case of COVID-19 infection that was not noticed at the time of hospitalization `The greatest weapon of the novel coronavirus is a stealth attack' -Warning to general hospitals-" (Japanese Association for Infectious Diseases website: http://www.kansensho.or.jp/uploads/files/topics/2019ncov/covid19_casereport_200403 _2.pdf). Note that the occurrence of pneumonia associated with novel coronavirus infection (COVID-19) was confirmed by simple chest computed tomography (CT). On the other hand, fever, heart palpitations, and so forth are listed as major symptoms of novel coronavirus infection (COVID-19). Taking such a background into account, the present aspect example may be configured to use data other than blood oxygen saturation data (e.g., body temperature data, heart rate data, etc.) as an evaluation index for pneumonia associated with novel coronavirus infection (COVID-19). The same may apply to other types of infectious diseases. Is should be noted that, as mentioned in the case report mentioned above, care must be taken when handling data that are affected by other factors, such as body temperature data of a patient who takes antipyretic analgesics.

According to the following document, it is known that continuation of hypoxemia increases the tortuosity of a fundus blood vessel: Irena Tsui et al. "Retinal Vascular Patterns in Adults with Cyanotic Congenital Heart Disease", Journal Seminars in Ophthalmology, Volume 24, 2009, Issue 6. Therefore, it is supposed that a running pattern of a blood vessel understood from fundus image data can change as hypoxemia progresses. In consideration of such a background, the present aspect example may be configured to use fundus image data, which has been acquired by an ophthalmic imaging apparatus in the data acquiring unit 10, as an evaluation index for hypoxemia associated with novel coronavirus infection (COVID-19). The same may apply to other types of infectious diseases.

According to the following document, it is known that the color tone of a living tissue changes due to hypoxemia: Adrian Spiteri "The blue patient" (http://dx.doi.org/10.1136/emerged-2016-205729). This phenomenon is caused by the fact that oxyhemoglobin in arterial blood decreases due to a decrease in blood oxygen concentration, resulting in the appearance of a color similar to that of venous blood. Considering the fact that changes in color tones are remarkable in body parts such as the tongue and lips in which capillaries can be seen through, it is supposed that the color tone of the eye fundus also changes significantly. Taking such a background into account, the present aspect example may be configured to use color fundus image data, which has been acquired by an ophthalmic imaging apparatus in the data acquiring unit 10, as an evaluation index for hypoxemia associated with novel coronavirus infection (COVID-19). The same may apply to other types of infectious diseases.

In an early stage of novel coronavirus infection (COVID-19) (i.e., if the degree of severity of novel coronavirus infection (COVID-19) is mild), fever, dehydration, and hypoxemia may lead to sympathetic dominance (sympathetic nervous system dominance), resulting in increased heart rate and cardiac output, a stronger heartbeat, and increased blood pressure. According to the following document, in a study of 137 patients with novel coronavirus infection (COVID-19), the first symptom (initial symptom) in 7.3% of patients was palpitations: Kui Liu et al. "Clinical characteristics of novel coronavirus cases in tertiary hospitals in Hubei Province", Chinese Medical Journal, 2020; Vol (No), (DOI: 10. 1097/CM9. 0000000000000744).

In the case where an increase in arterial blood flow rate is exhibited in this way, it is considered that the waveform of an arterial blood flow tends to be "Large & bounding" among the five pulse types described in the following document: Zhaopeng Fan et al. "Pulse Wave Analysis", Advanced Biomedical Engineering, Dr. Gaetano Gargiulo (Ed), 2011 (ISBN: 978-953-307-555-6).

On the other hand, according to the following document, it is known that as the severity of novel coronavirus infection (COVID-19) increases, systemic inflammation causes blood vessels to dilate (vasodilation) and therefore blood pressure to decrease as well as inflammation and hypoxia damage myocardial cells (heart muscle cells) and therefore cardiac functions deteriorate: Yasemin Saplakoglu, "The mysterious connection between the coronavirus and the heart", LIVE SCIENCE (https://www.livescience.com/how-coronavirus-affects-heart.html). Then, the myocardial contractile ability (the ability of heart muscles to contract) becomes reduced, resulting in an inability to pump adequately, a weakening of the heartbeat, and a decrease in cardiac output (heart output). In this case, it is considered that the shape of the waveform of an arterial blood flow tends to be "small & weak" among the five waveform types mentioned above. If this is the case, oxygen saturation (SpO2) is considered to be even lower.

In consideration of such a background, the present aspect example may be configured to use ocular blood flow data and/or blood oxygen saturation data, which has been acquired respectively by an ocular blood flow measurement apparatus in the data acquiring unit 10 and by a blood oxygen measurement apparatus in the data acquiring unit 10, as an evaluation index for symptoms associated with novel coronavirus infection (COVID-19). The same may apply to other types of infectious diseases.

According to the following document, it is known that an abnormal cardiac function is reflected in the shape of waveforms representing time series changes in the blood flow velocity of a retinal artery acquired using an optical coherence tomography blood flowmeter: Kana Minamide et al., "What can be seen from fundus blood flow measurement using optical coherence tomography (OCT)", Frontiers in computational science connected by 23 advanced cases: Understanding and predicting things by calculation (Kindai Kagaku-sha), Chapter 21, 2020. For example, FIG. 21.6 (p. 255) of the same document shows a comparison of retinal artery waveforms of a patient with aortic valve stenosis (before and after treatment) and a retinal artery waveform of a normal person. Thus, the shape of a retinal artery waveform changes due to a disease that causes abnormalities in cardiac pumping. Similarly, changes in a cardiac function associated with aggravation of an infectious disease are thought to appear in the shape of a waveform for one heartbeat acquired using an optical coherence tomography blood flowmeter. For example, a time delay from an initial phase of a heartbeat to a peak phase is indicative of a reduction in the contractile function of a heart. In addition, a decrease in the area under a waveform for one heartbeat is indicative of a decrease in a cardiac output.

Considering such a background, the present aspect example may be configured to use ocular blood flow data, which has been acquired by an ocular blood flow measurement apparatus (e.g., optical coherence tomography blood flowmeter) in the data acquiring unit 10, as an evaluation index for cardiac dysfunction (circulatory dysfunction) associated with novel coronavirus infection (COVID-19). The same may apply to other types of infectious diseases.

According to the following document, it is known that a blood flow condition inside an intracerebral artery (middle cerebral artery) changes due to an infectious disease: Haring HP et al. "Time course of cerebral blood flow velocity in central nervous system infections. A transcranial Doppler sonography study.", Arch Neurol. 1993 Jan; 50(1): 98-101. Novel coronavirus infection (COVID-19) can cause central nervous system symptoms and may produce a condition (pathology) similar to encephalitis (viral meningitis). In this case, systemic inflammation is thought to cause an increased systemic blood flow, resulting in an increase in a cerebral blood flow. Considering the fact that the ophthalmic artery branches from the same blood vessel as the middle cerebral artery, it is highly likely that a change in a blood flow also occurs in the ophthalmic artery and also the retinal artery beyond it. Therefore, a change in a cerebral blood flow associated with worsening of an infectious disease may be detected from a parameter such as a blood flow velocity value or a blood flow rate value obtained by the use of an optical coherence tomography blood flowmeter.

Taking such a background into account, the present aspect example may be configured to use ocular blood flow data, which has been acquired by an ocular blood flow measurement apparatus (e.g., optical coherence tomography blood flowmeter) in the data acquiring unit 10, as an evaluation index for an abnormal cerebral blood flow associated with novel coronavirus infection (COVID-19). The same may apply to other types of infectious diseases.

In regard to auscultatory sound data, it may be used as an evaluation index for pneumonia associated with novel coronavirus infection (COVID-19) by, for example, implementing the technique or technology disclosed in the following document: Japanese Unexamined Patent Application Publication (Translation of PCT Application) No. 2018-516616 (International Publication No. WO 2016/166318). The same may apply to other types of infectious diseases.

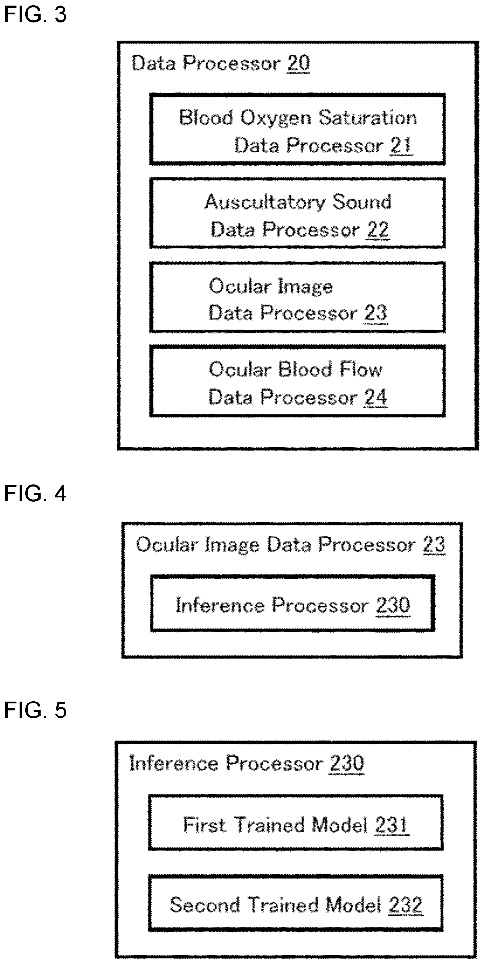

The data processor 20 is configured, for example, to perform data processing based on any of the above knowledge in order to detect a certain conditional change in a circulatory system associated with a certain infectious disease. FIG. 3 shows an example of the configuration of the data processor 20 of the present aspect. The data processor 20 of the present example includes the blood oxygen saturation data processor 21, the auscultatory sound data processor 22, the ocular image data processor 23, and the ocular blood flow data processor 24. The data processor 20 may be configured to generate a final output on the basis of outputs from two or more of these processors 21 to 24.

The blood oxygen saturation data processor 21 may be configured to perform diagnostic inference regarding a state or condition (change in a state or condition) of a circulatory system associated with an infectious disease by, for example, executing processing of blood oxygen saturation data, which is stored in the blood oxygen saturation data part 110 of the data configuration 100 shown in FIG. 2, by the use of a processor configured to operate in accordance with a program created based at least on the above knowledge on blood oxygen saturation. For example, the blood oxygen saturation data processor 21 may perform diagnostic inference by comparing an oxygen saturation value shown by the blood oxygen saturation data with a predetermined threshold value.

Further, the blood oxygen saturation data processor 21 may be configured to perform diagnostic inference regarding a state or condition (change in a state or condition) of a circulatory system associated with an infectious disease by, for example, executing processing of blood oxygen saturation data, which is stored in the blood oxygen saturation data part 110, using a trained model constructed by machine learning based at least on the above knowledge on blood oxygen saturation. This machine learning may be performed using training data that includes, for example, clinically collected blood oxygen saturation data and corresponding diagnostic result data. This diagnostic result data may be determined, for example, by a doctor or another inference model (trained model) on the basis of relevant blood oxygen saturation data. The trained model constructed in this way is configured to receive an input of blood oxygen saturation data recorded in the blood oxygen saturation data part 110 and to output inferred diagnostic data regarding a condition (conditional change) of a circulatory system associated with an infectious disease.

The auscultatory sound data processor 22 may be configured to perform diagnostic inference regarding a state or condition (change in a state or condition) in a circulatory system associated with an infectious disease by, for example, executing processing of auscultatory sound data by the use of a processor configured to operate in accordance with a program created based at least on the above knowledge on auscultatory sounds such as breath sounds and/or heart sounds. Here, the auscultatory sound data to be processed is recorded in the auscultatory sound data part 120 of the data configuration 100 shown in FIG. 2 and may be, for example, lung sound data recorded in the lung sound data part 121. For example, the auscultatory sound data processor 22 may be configured to perform diagnostic inference on the basis of a characteristic (feature, property) of a waveform shown in the auscultatory sound data, that is, on the basis of a sound profile of the auscultatory sound data.

Further, the auscultatory sound data processor 22 may be configured to perform diagnostic inference regarding a state or condition (change in a state or condition) in a circulatory system associated with an infectious disease by, for example, executing processing of auscultatory sound data using a trained model constructed by machine learning based at least on the above knowledge on auscultatory sounds such as breath sounds and/or heart sounds. Here, the auscultatory sound data to be processed is recorded in the auscultatory sound data part 120, and may include lung sound data recorded in the lung sound data part 121. This machine learning may be conducted using training data that includes, for example, clinically collected auscultatory sound data and corresponding diagnostic result data. This diagnostic result data is determined, for example, by a doctor or another inference model (trained model) based on relevant auscultatory sound data. The trained model constructed in this way is configured to receive an input of auscultatory sound data recorded in the auscultatory sound data part 120 (e.g., lung sound data recorded in the lung sound data part 121), and to output inferred diagnostic data regarding a condition (conditional change) of a circulatory system associated with an infectious disease.

The ocular image data processor 23 may be configured to perform diagnostic inference regarding a state or condition (change in a state or condition) in a circulatory system associated with an infectious disease by, for example, executing processing of ocular image data by the use of a processor configured to operate in accordance with a program created based at least on the above knowledge on an ocular image such as a fundus image. Here, the ocular data to be processed is recorded in the ocular image data part 130 of the data configuration 100 shown in FIG. 2 and may include either one of or both fundus image data recorded in the fundus image data part 131 and color fundus image data recorded in the color fundus image data part 132. For example, the ocular image data processor 23 may be configured to perform diagnostic inference based on a characteristic (feature, property) of ocular image data such as blood vessel tortuosity, color information, and so forth.

In some examples, the ocular image data processor 23 may be configured to perform diagnostic inference regarding a state or condition (change in a state or condition) in a circulatory system associated with an infectious disease by, for example, executing processing of ocular image data using a trained model constructed by machine learning based at least on the above knowledge on an ocular image such as a fundus image. Here, the ocular image data to be processed is recorded in the ocular image data part 130, such as either one of or both fundus image data recorded in the fundus image data part 131 and color fundus image data recorded in the color fundus image data part 132. This machine learning may be conducted using training data that includes, for example, clinically collected ocular image data and corresponding diagnostic result data. This diagnostic result data is determined, for example, by a doctor or another inference model (trained model) based on relevant ocular image data. The trained model constructed in this way is configured to receive an input of ocular image data recorded in the ocular image data part 130 (e.g., fundus image data recorded in the fundus image data part 131 and/or color fundus image data recorded in the color fundus image data part 132), and to output inferred diagnostic data regarding a condition (conditional change) of a circulatory system associated with an infectious disease.

An example of the ocular image data processor 23 configured using machine learning is shown in FIG. 4. The ocular image data processor 23 of the present example includes the inference processor 230. As described above, the inference processor 230 may be configured to execute inference processing that derives information on a conditional change in a circulatory system from ocular image data acquired from a patient by the data acquiring unit 10, by using a trained model constructed by machine learning using training data that includes clinical data such as ocular image data and diagnostic result data.

By conducting machine learning (supervised learning) based on such training data, a trained model (inference model) is created which is configured to receive an input of ocular image data acquired from a patient by the data acquiring unit 10 and to output inferred diagnostic data on a conditional change in a circulatory system.

The inference processor 230 includes the trained model obtained in this way, and is configured to input ocular image data acquired from a patient by the used of the data acquiring unit 10 into the trained model, and to send resulting inferred diagnostic data output from the trained model to the output unit 30.

Machine learning algorithms that can be used for some aspect examples are not limited to supervised learning, and may be any types of algorithms such as unsupervised learning, semi-supervised learning, reinforcement learning, transduction, and multi-task learning. A combination of any two or more algorithms may also be employed.

Methods and techniques of machine learning that can be used for some aspect examples may be freely selected, and may be any methods and techniques such as neural network, support vector machine, decision tree learning, association rule learning, genetic programming, clustering, Bayesian network, feature learning, representation learning, and extreme learning machine. A combination of any two or more methods and techniques may also be employed.

An example of the configuration of the inference processor 230 is shown in FIG. 5. The inference processor 230 of the present example includes the first trained model 231 and the second trained model 232.

The first trained model 231 is constructed by machine learning using training data that includes fundus image data and diagnostic result data. For example, the first trained model 231 includes a convolutional neural network. The convolutional neural network includes, for example, an input layer, a convolutional layer, a pooling layer, a fully connected layer, and an output layer. Fundus image data is input into the input layer, and the convolutional layer applies filtering (convolution) to the fundus image data input, thereby creating a feature map with regard to tortuosity of blood vessels. The pooling layer executes data compression while retaining the features obtained by the convolutional layer, and the fully connected layer extracts characteristic findings (feature findings, property findings) from all the data obtained by the pooling layer and makes a determination (judgment). The output layer outputs the data obtained by the fully connected layer. By inputting fundus image data acquired from a patient by the data acquiring unit 10 into the first trained model 231, information regarding a conditional change in a circulatory system with the tortuosity state of blood vessels taken into account is generated. By employing the first trained model 231 configured in this manner, the data processor 20 is capable of detecting a conditional change in a circulatory system on the basis of a running pattern of blood vessels depicted in fundus image data of a patient.

It should be noted that features related to a blood vessel are not limited to running patterns thereof. For example, a change in a blood vessel diameter (dilation and contraction of a blood vessel) may be taken into consideration to obtain information on a conditional change in a circulatory system. Blood vessel diameter may be calculated, for example, by analyzing fundus image data or optical coherence tomography image data. In order to implement blood vessel diameter measurement, any known techniques or methods can be employed, for example those described in the following documents: Japanese Unexamined Patent Application Publication No. 2016-43155, and Japanese Unexamined Patent Application Publication No. 2020-48730.

The second trained model 232 is constructed by machine learning using training data that includes color fundus image data and diagnostic result data. For example, the second trained model 232 includes a convolutional neural network. The convolutional neural network includes, for example, an input layer, a convolutional layer, a pooling layer, a fully connected layer, and an output layer. Color fundus image data is input into the input layer, and the convolutional layer creates a feature map with regard to color information (e.g., R value, G value, B value) by applying filtering (convolution) to the color fundus image data input. The pooling layer executes data compression while retaining the features obtained by the convolutional layer, and the fully connected layer extracts characteristic findings (feature findings, property findings) from all the data obtained in the pooling layer and makes a determination (judgment). The output layer outputs the data obtained by the fully connected layer. By inputting color fundus image data acquired from a patient by the data acquiring unit 10 into the second trained model 232, information regarding a conditional change in a circulatory system with the color tone of an eye fundus taken into account is generated. By employing the second trained model 232 configured in this manner, the data processor 20 is capable of detecting a conditional change in a circulatory system on the basis of color information from color fundus image data of a patient.

A trained model configured to process data of a type different from ocular image data may also have a configuration similar to that described above. In the case of processing time series data such as waveform data, follow-up data, video data, or audio data, a trained model for this processing may include a recurrent neural network. Training data used for machine learning may include data generated from clinical data by a computer. Machine learning may include transfer learning.

The ocular blood flow data processor 24 may be configured to execute diagnostic inference regarding a state or condition (change in a state or condition) in a circulatory system associated with an infectious disease by, for example, executing processing of ocular blood flow data by the use of a processor configured to operate in accordance with a program created based at least on the above knowledge on ocular blood flow such as fundus blood flow. Here, the ocular blood flow data to be processed is recorded in the ocular blood flow data part 140 of the data configuration 100 shown in FIG. 2, and may include one or more of the following types of data: waveform data recorded in the waveform data part 142, blood flow velocity data recorded in the blood flow velocity data part 143, and blood flow rate data recorded in the blood flow rate data part 144. In some examples, the ocular blood flow data processor 24 may be configured to perform diagnostic inference based on a characteristic (feature, property) of ocular blood flow data such as a parameter that represents a feature of a waveform.

In some examples, the ocular blood flow data processor 24 may be configured to perform diagnostic inference regarding a state or condition (change in a state or condition) in a circulatory system associated with an infectious disease by, for example, executing processing of ocular blood flow data using a trained model constructed by machine learning based at least on the above knowledge on ocular blood flow such as fundus blood flow. Here, the ocular blood flow data to be processed is recorded in the ocular blood flow data part 140, and may include one or more of the following types of data: waveform data recorded in the waveform data part 142, blood flow velocity data recorded in the blood flow velocity data part 143, and blood flow rate data recorded in the blood flow rate data part 144. This machine learning may be conducted using training data that includes, for example, clinically collected ocular blood flow data and corresponding diagnostic result data. This diagnostic result data is determined, for example, by a doctor or another inference model (trained model) based on relevant ocular blood flow data. The trained model constructed in this way is configured to receive an input of ocular blood flow data recorded in the ocular blood flow data part 140 (e.g., at least one of: waveform data recorded in the waveform data part 142, blood flow velocity data recorded in the blood flow velocity data part 143, and blood flow rate data recorded in the blood flow rate data part 144), and to output inferred diagnostic data regarding a condition (conditional change) of a circulatory system associated with an infectious disease.

In the case where fundus blood flow data includes waveform data, the data processor 20 may be configured to detect a conditional change in a circulatory system based on this waveform data. This detection process typically takes into account a parameter that shows a characteristic (feature, property) of the waveform.

In the case where fundus blood flow data includes two or more pieces of waveform data respectively acquired from a patient in two or more different time periods (e.g., in the case where two or more pieces of waveform data have been acquired for the purpose of follow-up), the data processor 20 may detect a conditional change in a circulatory system by comparing the two or more pieces of waveform data. This detection process typically takes into account a change in a parameter that shows a characteristic (feature, property) of a waveform.

In the case where fundus blood flow data includes either one of or both blood flow velocity data and blood flow rate data for a fundus artery of a patient, the data processor 20 may detect a conditional change in a circulatory system based on either one of or both the blood flow velocity data and the blood flow rate data. This detection process typically takes into account the magnitude of the values thereof.

In the case where fundus blood flow data includes two or more pieces of blood flow velocity data respectively acquired from a patient in two or more different time periods (e.g., in the case where two or more pieces of blood flow velocity data have been acquired for the purpose of follow-up), the data processor 20 may detect a conditional change in a circulatory system by comparing the two or more pieces of blood flow velocity data. This detection process typically takes into account a change in the value of blood flow velocity.

In the case where fundus blood flow data includes two or more pieces of blood flow rate data respectively acquired from a patient in two or more different time periods (e.g., in the case where two or more pieces of blood flow rate data have been acquired for the purpose of follow-up), the data processor 20 may detect a conditional change in a circulatory system by comparing the two or more pieces of blood flow rate data. This detection process typically takes into account a change in the value of blood flow rate.

Similar processes can be employed in the cases of processing other types of data (e.g., body temperature data, heart rate data, blood pressure data, etc.). More specifically, in such cases, processing can be performed by the use of a processor configured to operate in accordance with a program created based at least on corresponding knowledge (relevant knowledge) and/or by the use of a trained model constructed by machine learning based at least on corresponding knowledge (relevant knowledge).

According to the data processor 20 of the present aspect example that has such a configuration, any of the following events (phenomena) can be detected as a conditional change in a circulatory system associated with an infectious disease: an onset of pneumonia, a change in a condition of pneumonia (becoming severer, becoming milder, becoming asymptomatic), an onset of hypoxemia, a change in a condition of hypoxemia (becoming severer, becoming milder, becoming asymptomatic), and a change in a condition of cerebral blood flow (becoming severer, becoming milder, becoming asymptomatic). The data processor 20 may be configured to be capable of detecting any other types of conditional changes associated with an infectious disease, and also may be configured to be capable of detecting any types of conditional changes (regardless of the connection with an infectious disease).

In addition, an item (e.g., disease, symptom, etc.) on which diagnostic inference is executed by the data processor 20 may be freely selected. For example, the data processor 20 may be configured to perform inference processing regarding pneumonia. More specifically, the data processor 20 may be configured to perform the following types of inference processing: inference processing for calculating a probability that a patient has pneumonia (pneumonia contraction probability), inference processing for calculating a probability that a patient has an infectious disease associated with pneumonia (infectious disease contraction probability), inference processing for determining a severity of pneumonia in a patient (pneumonia severity), and inference processing for determining a severity of infectious disease associated with pneumonia in the patient (infectious disease severity).

The infectious disease associated with pneumonia may be, for example, novel coronavirus infection (COVID-19). Further, the severity of the infectious disease associated with pneumonia may be related to freely selected symptoms such as cytokine storm, fever, conjunctival hyperemia (conjunctival congestion), nasal congestion, headache, cough, sore throat, sputum, bloody sputum, malaise, fatigue, shortness of breath, nausea, vomiting, diarrhea, myalgia, joint pain, chills, and so forth. In addition, the severity of the infectious disease associated with pneumonia may be related to any underlying medical conditions, application of dialysis, administration of specific medications, and so forth. Examples of the underlying medical conditions include diabetes, heart failure, and respiratory diseases such as chronic obstructive pulmonary disease (COPD). Examples of the specific medications include immunosuppressants (immunosuppressive drugs, immunosuppressive agents), and anticancer drugs.

The output unit 30 is configured to output a result of processing performed by the data processor 20. The mode or aspect of output processing may be freely selected, and may be any of transmission, display, recording, and printing. Information output by the output unit 30 may be the result of the processing executed by the data processor 20 (e.g., a result obtained by the detection of a conditional change in a circulatory system associated with an infectious disease), may be information including the result of the processing, or may be information generated by applying further processing to the result of the processing. For example, the medical system 1 may further include a report creating unit (not shown in the drawings) that is configured to generate a report based on a result of detection of a conditional change in a circulatory system obtained by the data processor 20. If this is the case, the output unit 30 may output the report generated in this way.

A configuration example of the output unit 30 is shown in FIG. 1. The output unit 30 of the present example includes the transmitter 31. The transmitter 31 is configured to transmit a result of processing performed by the data processor 20 to the doctor's computer terminal 3. The doctor's computer terminal 3 is located remotely from the data acquiring unit 10.

Here, the transmission from the output unit 30 to the doctor's computer terminal 3 may be direct transmission or indirect transmission. The direct transmission is a mode or aspect of transmitting a result of the processing (e.g., a detection result, a report, etc.) from the output unit 30 to the doctor's computer terminal 3. On the other hand, the indirect transmission is a mode or aspect of transmitting a result of the processing to an apparatus or device (e.g., a server, a database, etc.) other than the doctor's computer terminal 3 and then providing the result of the processing to the doctor's computer terminal 3 from this apparatus or device.

By employing the configuration, as described thus far, in which the doctor's computer terminal 3 is placed at a remote location from the data acquiring unit 10 and in which a result of processing (or information generated from a result of processing) acquired by the data processor 20 based on data acquired from a patient by the data acquiring unit 10 is provided to the doctor's computer terminal 3, social distance between the doctor (healthcare worker) and the patient can be maintained, and therefore the risk of infection to the doctor (healthcare worker) can be reduced.

### < Usage mode of medical system >

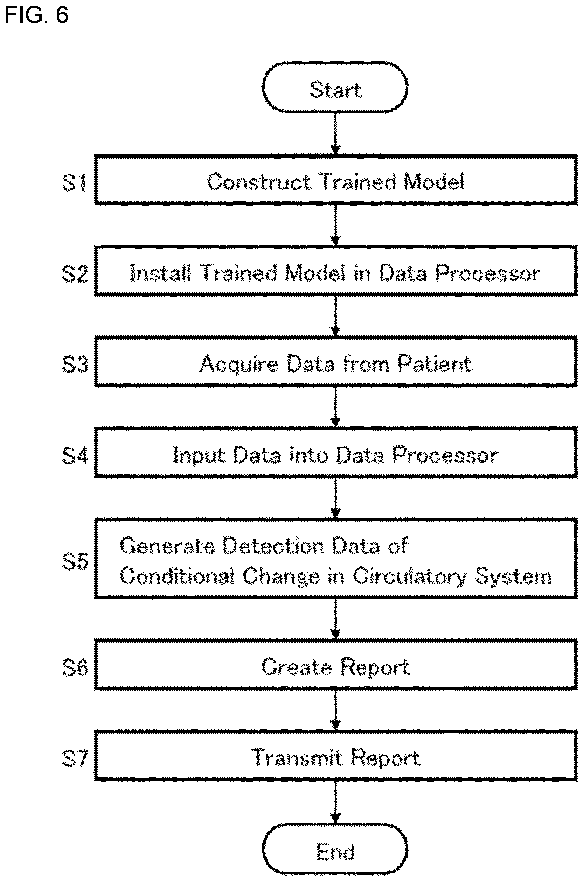

An example of a usage mode of the medical system 1 according to the present aspect example will be described with reference to the flowchart shown in FIG. 6.

### (S1: Construct trained model)

In preparation for putting the medical system 1 into implementation, a trained model to be used by the data processor 20 is constructed. Note that processing performed at this stage may be updating of a trained model that has already been used, that is, adjustment or updating of parameters in a trained model that has already been used.

### (S2: Install trained model in data processor)

In further preparation for putting the medical system 1 into implementation, the trained model constructed in the step S1 is installed in the data processor 20. In this process, for example, the trained model constructed in the step S1 is transmitted to the medical system 1 through a communication line.

### (S3: Acquire data from patient)

A subject may be, for example, a patient with a confirmed diagnosis (definitive diagnosis) of novel coronavirus infection (COVID-19) or a patient with suspected novel coronavirus infection (COVID-19). The data acquiring unit 10 of the medical system 1 acquires data of a predetermined item from a patient. In the present aspect, the data acquiring unit 10 acquires from the patient two or more types of data among blood oxygen saturation data, auscultatory sound data, ocular image data, and ocular blood flow data. In addition to these types of data, freely selected types of data may be acquired from the patient, such as body temperature data, heart rate data, blood pressure data, ocular characteristic data, and so forth.

One or more of the examinations carried out in the present process may be a remote examination(s) using the operation device 2.

The acquired data is recorded, for example, in conformity with the data configuration 100 in FIG. 2. With this, a data package for this patient is obtained.

As described thus far, two or more types of data are acquired from the patient in the present aspect. Note that the timings (time points) of acquisitions of the two or more types of data may be freely determined. For example, the second data may be acquired after acquisition of the first data, the first data may be acquired after acquisition of the second data, or the first data and the second data may be acquired in parallel or simultaneously.

In addition, the difference (time difference) between the acquisition timings (time points) of two or more types of data may also be freely determined. For example, in the case where at least fundus blood flow data and heart rate data are acquired, since there is a time lag between the state (condition) of fundus blood flow and the state (condition) of heart rate, there is no need to acquire both fundus blood flow data and heart rate data at the same time, and, for example, about 10 minutes of time difference between their acquisitions may be allowed. However, it is considered desirable that a condition affecting a blood circulation state, such as posture, be the same for both data acquisitions. The same applies for any condition that can affect other examination parameters, such as diet, time slot, drug administration, and so forth.

### (S4: Input data into data processor)

The data acquired in the step S3 is sent to the data processor 20. At least part of the data that has been input into the data processor 20 is input into the trained model constructed in the step S1.

### (S5: Generate detection data of conditional change in circulatory system)

The data processor 20 executes processing of the data input in the step S4 to detect a conditional change in a circulatory system associated with an infectious disease. As a result of this processing, the data processor 20 generates, for example, any of the following types of data: data showing an onset of pneumonia, data showing a change in the condition of pneumonia, data showing an onset of hypoxemia, data showing a change in the condition of hypoxemia, and data showing a change in the condition of cerebral blood flow. The data generated in the present process is referred to as detection data.

### (S6: Create report)

The medical system 1 (the report creating unit mentioned above, which is not shown in the drawings) creates a report based on the detection data generated in the step S5.

### (S7: Transmit report)

The transmitter 31 of the output unit 30 transmits the report created in the step S6 to the doctor's computer terminal 3 that is located remotely from the data acquiring unit 10. Alternatively, the transmitter 31 of the output unit 30 transmits the report to a computer that is capable of providing information to the doctor's computer terminal 3. The doctor's computer terminal 3 is not limited to a computer used by a doctor, and may be a computer used by a healthcare worker other than a doctor (healthcare worker's computer terminal).

According to the medical system 1 of the present aspect example, it becomes possible to keep social distances between healthcare workers and patients, and the risks of healthcare workers getting infected from patients can be reduced. Furthermore, since the medical system 1 is configured to automatically perform diagnostic inference based on two or more types of data among blood oxygen saturation data, auscultatory sound data, ocular image data, and ocular blood flow data, a symptom associated with an infectious disease and an aggravation sign can be detected with higher precision than conventional techniques.

Examinations conducted by a medical system according to some aspect examples are non-invasive. For example, all of the following apparatuses or devices, which are examples of examination apparatuses that can be used in the medical system 1, can acquire data from a patient in a noninvasive manner: a pulse oximeter, an electronic stethoscope, an optical coherence tomography apparatus, a fundus camera (of non-mydriatic type), a slit lamp microscope, a scanning laser ophthalmoscope, laser speckle flowgraphy apparatus, a thermometer, a heart rate meter, an electrocardiogram meter, a blood flow meter, a respiratory monitoring system, a blood pressure meter, an ocular refraction measurement apparatus, a tonometer, a specular microscope, a wavefront analyzer, a visual acuity test apparatus, a perimeter, a microperimeter, an ocular axial length measurement apparatus, an electroretinography apparatus, a binocular visual function examination apparatus, a color vision examination apparatus. Non-invasive examination apparatuses that can be used in the medical system of aspect examples are not limited to these. Note that one or more of the examination apparatuses may be invasive such as a mydriatic-type fundus camera.

### < Medical information processing apparatus >

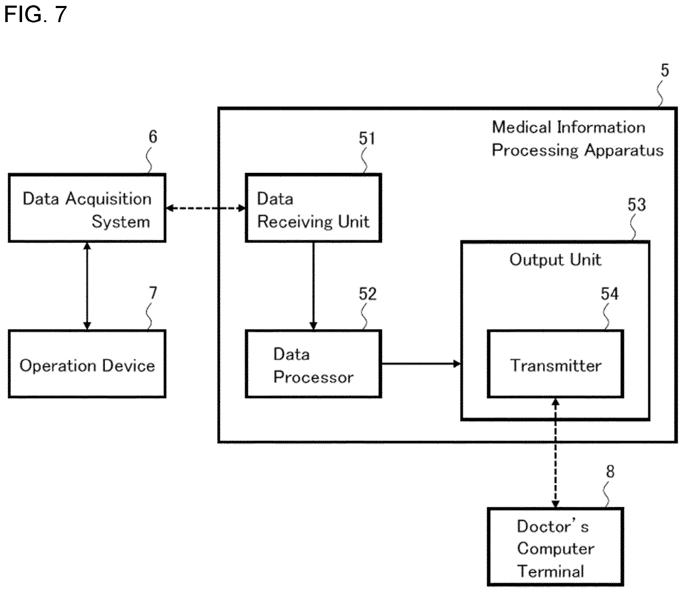

An example of a medical information processing apparatus according to some aspect examples will be described. An example of the medical information processing apparatus 5 shown in FIG. 7 includes the data receiving unit 51, the data processor 52, and the output unit 53.

The data acquisition system 6, the operation device 7, and the doctor's computer terminal 8 are provided exterior to the medical information processing apparatus 5 of the present aspect.

The data acquisition system 6 is configured to acquire from a patient two or more types of data among blood oxygen saturation data, auscultatory sound data, ocular image data, and ocular blood flow data. The data acquisition system 6 may also be configured to further acquire from the patient either one of or both body temperature data and heart rate data.

At least part of the configuration of the data acquisition system 6 may be the same as at least part of the data acquiring unit 10 of the medical system 1 described above. Any matters and items described for the data acquiring unit 10 can be combined with the data acquisition system 6. The data acquired by the data acquisition system 6 may, for example, be recorded in conformity with the data configuration 100 shown in FIG. 2.

The operation device 7 is used by a healthcare worker to perform remote operation of the data acquisition system 6 (examination apparatus). The operation device 7 includes, for example, a computer and an operation panel. At least part of the configuration of the operation device 7 may be the same as at least part of the operation device 2 described above. Any matters and items described for the operation device 2 can be combined with the operation device 7.

The doctor's computer terminal 8 is located at a remote location from the data acquisition system 6. At least part of the configuration of the doctor's computer terminal 8 may be the same as at least part of the doctor's computer terminal 3 described above. Any matters and items described for the doctor's computer terminal 3 can be combined with the doctor's computer terminal 8.

The data receiving unit 51 is configured to receive two or more types of data among blood oxygen saturation data, auscultatory sound data, ocular image data, and ocular blood flow data that have been acquired from the patient. In the example shown in FIG. 7, data is input from the data acquisition system 6 into the data receiving unit 51, but the manner or mode of data input is not limited to this. For example, data acquired by the data acquisition system 6 may be stored in a database or the like, and then the data may be input into the data receiving unit 51 from this database. The data receiving unit 51 may include, for example, a communication device (telecommunication equipment) for connecting to a communication line, a drive device that reads out data recorded on a recording medium, and so forth.

The data processor 52 is configured to execute processing of the two or more types of data received by the data receiving unit 51 in order to detect a conditional change in a circulatory system associated with an infectious disease. At least part of the configuration of the data processor 52 may be the same as at least part of the data processor 20 of the medical system 1 described above. Any matters and items described for the data processor 20 can be combined with the data processor 52.

The output unit 53 is configured to output a result of the processing carried out by the data processor 52. The output unit 53 of the present example includes the transmitter 54. The transmitter 54 is configured to transmit a result of the processing carried out by the data processor 52 to the doctor's computer terminal 8 that is located remotely from the data acquisition system 6. At least part of the configuration of the output unit 53 may be the same as at least part of the output unit 30 of the medical system 1 described above. Any matters and items described for the output unit 30 can be combined with the output unit 53. Similarly, at least part of the configuration of the transmitter 54 may be the same as at least part of the transmitter 41 of the medical system 1 described above, and any matters and items described for the transmitter 41 can be combined with the transmitter 54.

Any matters and items described for the medical system 1 described above can be combined with the medical information processing apparatus 5.

According to the medical information processing apparatus 5 described herein, it becomes possible to keep social distances between healthcare workers and patients, and the risks of healthcare workers getting infected from patients can be reduced. Furthermore, since the medical information processing apparatus 5 is configured to automatically perform diagnostic inference based on two or more types of data among blood oxygen saturation data, auscultatory sound data, ocular image data, and ocular blood flow data, a symptom associated with an infectious disease and an aggravation sign can be detected with higher precision than conventional techniques.

### EXPLANATION OF REFERENCE CHARACTERS

- 1: Medical system
- 2: Operation device
- 3: Doctor's computer terminal
- 10: Data acquiring unit
- 20: Data processor
- 21: Blood oxygen saturation data processor
- 22: Auscultatory sound data processor
- 23: Ocular image data processor
- 230: Inference processor
- 231: First trained model
- 232: Second trained model
- 24: Ocular blood flow data processor
- 30: Output unit
- 31: Transmitter
- 100: Data configuration
- 110: Blood oxygen saturation data part
- 120: Auscultatory sound data part
- 130: Ocular image data part
- 140: Ocular blood flow data part

## Claims

1. A medical system comprising:
a data acquiring unit configured to acquire from a patient two or more types of data among blood oxygen saturation data, auscultatory sound data, ocular image data, and ocular blood flow data; and
a data processor configured to process the two or more types of data acquired by the data acquiring unit to detect a conditional change in a circulatory system associated with an infectious disease.

2. The medical system according to claim 1, wherein the conditional change in the circulatory system includes one or more of an onset of pneumonia, a change in a condition of pneumonia, an onset of hypoxemia, a change in a condition of hypoxemia, and a change in a condition of cerebral blood flow.

3. The medical system according to claim 1 or 2, wherein the ocular image data includes fundus image data depicting an eye fundus of the patient.

4. The medical system according to claim 3, wherein the data processor is configured to detect the conditional change in the circulatory system based on a running pattern of a blood vessel depicted in the fundus image data.

5. The medical system according to claim 4, wherein the fundus image data includes one or more types of image data among fundus camera image data, slit lamp image data, optical coherence tomography image data, and scanning laser image data.

6. The medical system according to claim 1 or 2, wherein the ocular image data includes color fundus image data depicting an eye fundus of the patient.

7. The medical system according to claim 6, wherein the data processor is configured to detect the conditional change in the circulatory system based on color information in the color fundus image data.

8. The medical system according to claim 7, wherein the color fundus image data includes one or more types of image data among fundus camera image data, slit lamp image data, and scanning laser image data.

9. The medical system according to any of claims 1 to 8, wherein the data processor includes an inference processor configured to perform inference processing that derives information on the conditional change in the circulatory system from the ocular image data acquired by the data acquiring unit by using a trained model constructed by machine learning using training data that includes ocular image data and diagnostic result data.

10. The medical system according to any of claims 1 to 9, wherein the ocular blood flow data includes fundus blood flow data that represents blood flow dynamics in an eye fundus blood vessel of the patient.

11. The medical system according to claim 10, wherein
the fundus blood flow data includes waveform data that represents a time series change in blood flow velocity in an eye fundus artery of the patient, and
the data processor is configured to detect the conditional change in the circulatory system based on the waveform data.

12. The medical system according to claim 11, wherein
the fundus blood flow data includes two or more pieces of waveform data respectively acquired from the patient in two or more different time periods, and
the data processor is configured to detect the conditional change in the circulatory system by comparing the two or more pieces of waveform data.

13. The medical system according to any of claims 10 to 12, wherein
the fundus blood flow data includes either one of or both blood flow velocity data and blood flow rate data for the eye fundus artery of the patient, and
the data processor is configured to detect the conditional change in the circulatory system based on either one of or both the blood flow velocity data and the blood flow rate data.

14. The medical system according to any of claims 10 to 13, wherein the data acquiring unit includes an optical coherence tomography apparatus configured to perform scanning of an eye fundus of the patient to acquire the fundus blood flow data.

15. The medical system according to any of claims 1 to 14, wherein the auscultatory sound data includes lung sound data acquired from the patient by an electronic stethoscope.

16. The medical system according to any of claims 1 to 15, wherein the data acquiring unit is configured to further acquire either one of or both body temperature data and heart rate data from the patient.

17. The medical system according to any of claims 1 to 16, further comprising a transmitter configured to transmit information output from the data processor to a doctor's computer terminal located remotely from the data acquiring unit.

18. The medical system according to any of claims 1 to 17, further comprising an operation unit used for performing a remote operation of the data acquiring unit.

19. A medical information processing apparatus comprising:
a data receiving unit configured to receive two or more types of data among blood oxygen saturation data, auscultatory sound data, ocular image data, and ocular blood flow data acquired from a patient; and
a data processor configured to process the two or more types of data received by the data receiving unit to detect a conditional change in a circulatory system associated with an infectious disease.

20. The medical information processing apparatus according to claim 19, wherein the conditional change in the circulatory system includes one or more of an onset of pneumonia, a change in a condition of pneumonia, an onset of hypoxemia, a change in a condition of hypoxemia, and a change in a condition of cerebral blood flow.

21. The medical information processing apparatus according to claim 19 or 20, wherein the ocular image data includes fundus image data depicting an eye fundus of the patient.

22. The medical information processing apparatus according to claim 21, wherein the data processor is configured to detect the conditional change in the circulatory system based on a running pattern of a blood vessel depicted in the fundus image data.

23. The medical information processing apparatus according to claim 19 or 20, wherein the ocular image data includes color fundus image data depicting an eye fundus of the patient.

24. The medical information processing apparatus according to claim 23, wherein the data processor is configured to detect the conditional change in the circulatory system based on color information in the color fundus image data.

25. The medical information processing apparatus according to any of claims 19 to 24, wherein the data processor includes an inference processor configured to perform inference processing that derives information on the conditional change in the circulatory system from the ocular image data received by the data receiving unit by using a trained model constructed by machine learning using training data that includes ocular image data and diagnostic result data.

26. The medical information processing apparatus according to any of claims 19 to 25, wherein the ocular blood flow data includes fundus blood flow data that represents blood flow dynamics in an eye fundus blood vessel of the patient.

27. The medical information processing apparatus according to claim 26, wherein
the fundus blood flow data includes waveform data that represents a time series change in blood flow velocity in an eye fundus artery of the patient, and
the data processor is configured to detect the conditional change in the circulatory system based on the waveform data.

28. The medical information processing apparatus according to claim 27, wherein
the fundus blood flow data includes two or more pieces of waveform data respectively acquired from the patient in two or more different time periods, and
the data processor is configured to detect the conditional change in the circulatory system by comparing the two or more pieces of waveform data.

29. The medical information processing apparatus according to any of claims 26 to 28, wherein
the fundus blood flow data includes either one of or both blood flow velocity data and blood flow rate data for the eye fundus artery of the patient, and
the data processor is configured to detect the conditional change in the circulatory system based on either one of or both the blood flow velocity data and the blood flow rate data.

30. The medical information processing apparatus according to any of claims 19 to 29, wherein the auscultatory sound data includes lung sound data.

31. The medical information processing apparatus according to any of claims 19 to 30, wherein the data receiving unit further receives either one of or both body temperature data and heart rate data acquired from the patient.

32. The medical information processing apparatus according to any of claims 19 to 31, further comprising a transmitter configured to transmit information generated by the data processor to a doctor's computer terminal located remotely from at least one apparatus used to acquire the two or more types of data from the patient.
